# EUROPEAN PATENT APPLICATION

(11) **EP 3 808 377 A1**
(43) Date of publication of application: **21.04.2021**
(21) Application number: 19810577.7
(22) Date of filing: 31.05.2019
(51) Int. Cl.: A61K 47/44, A61K 9/10, A61K 45/00, A61K 47/12, A61K 47/14, A61K 47/24, A61K 47/46, C07K 5/08

(54) **PERCUTANEOUS ABSORPTION PREPARATION**

(30) Priority: 31.05.2018 JP 2018104440
(71) Applicant: Kyushu University, National University Corporation, Nishi-ku Fukuoka-shi Fukuoka 819-0395 (JP); Nissan Chemical Corporation, Tokyo 103-6119 (JP)
(72) Inventor: KITAOKA Momoko, Fukuoka-shi, Fukuoka 819-0395 (JP); GOTO Masahiro, Fukuoka-shi, Fukuoka 819-0395 (JP)
(74) Representative: dompatent von Kreisler Selting Werner - Partnerschaft von Patent- und Rechtsanwälten mbB
(86) International application number: PCT/JP2019/021685
(87) International publication number: WO 2019/230939

(57) **Abstract**

Provided is a technique for delivering a hydrophilic drug having a large molecular weight such as a protein through the skin into the body.

A formulation which is a transdermal formulation containing a hydrophilic drug and an oily base, characterized in that at least a part of the hydrophilic drug is present in a suspended state in the oily base without being dissolved.

SEQUENCE LISTING

<110>  NISSAN CHEMICAL CORPORATION
       KYUSHU UNIVERSITY, NATIONAL UNIVERSITY CORPORATION

<120>  PERCUTANEOUS ABSORPTION PREPARATION

<130>  FP4435PCT

<150>  JP 2018-104440
<151>  2018-05-31

<160>  1

<170>  PatentIn version 3.5

<210>  1
<211>  18
<212>  PRT
<213>  Artificial Sequence

<220>
<223>  FITC-labeled cow's milk allergy peptide


<220>
<221>  BINDING
<222>  (1)..(1)
<223>  FITC-labeled

<400>  1

Leu Leu Asp Ala Gln Ser Ala Pro Leu Arg Val Tyr Val Glu Glu Leu
1               5                   10                  15


Lys Pro

## Description

### TECHNICAL FIELD

The present invention relates to a transdermal formulation containing a hydrophilic drug, an oily base, and occasionally an oil-soluble percutaneous permeation enhancing substance, and a method for percutaneously absorbing the hydrophilic drug.

### BACKGROUND ART

In recent years, a percutaneous drug administration method in which a drug is administered through the skin to the whole body has attracted attention as an administration method of a drug, especially an alternative method to the oral administration method and the injection method. The dosage forms used in the percutaneous administration method include creams, sprays, patches and the like. These dosage forms are considered to be extremely excellent ones because these dosage forms enable a long term administration, an administration with no injection pain, and the like.

However, there is a layer having high hydrophobicity called the stratum corneum with a thickness of about 20 µm at the outermost layer of human skin, which has a role of preventing evaporation of water in the body as well as a role of inhibiting invasion of adventive species or molecules. Therefore, it has been known that, unless any special compound (for chemical promotion method) or device (for physical promotion method) is used, a material which can reach into the body through the skin is only a molecule having relatively high hydrophobicity or a molecule having a molecular weight of 500 Da or less. Accordingly, hydrophilic polymers including protein formulations such as vaccines and immunoglobulin formulations are, in particular, difficult to be percutaneously administrated.

As a means for solving such problems, a method to deliver the drug into the body by chemically permeating the stratum corneum barrier, for example, a method of adding a percutaneous permeation enhancer such as a terpene, a fatty acid, and an alcohol, and a method of using particles having a nano-size such as a liposome-like substance, and polylactic acid (PLA) particles, have been reported (for example, see Non-Patent Document 1). However, carriers comprising lipid bilayers or Pluronic can contain an aqueous drug solution and disperse it in an aqueous base, but there are problems that the drug will deteriorate during long-term storage and an inclusion rate of the drug is low.

To the contrary, in recent years, an oil fitting well to the stratum corneum has been proposed to be used as a base agent to produce some formulations, for example, a lyotropic liquid crystal (for example, see Non-Patent Document 2), lecithin organogel (for example, see Non-Patent Document 3), Inverse Micellar Sugar Glass (IMSG) nano particles (for example, see Non-Patent Document 4), Water-in-Oil (W/O) microemulsion (for example, see Non-Patent Document 5), Solid-in-Oil (S/O) nano-dispersion (for example, see Patent Document 1, Non-Patent Document 6) and the like.

However, even in the system in which an oil is used as a base agent, when the system contains water or alcohol in the same manner as the above-mentioned lyotropic liquid crystal, lecithin organogel and W/O emulsion, there is a problem that the inclusion rate of the drug largely depends on its solubility in the water or alcohol, in addition to the same problems as in the aqueous base.

On the other hand, in a method using an emulsion of IMSG nano particles or S/O nano particles produced by freeze-drying, water is not contained in the final product but there is a problem that the usable surfactant or oily base is limited.

### PRIOR ART DOCUMENTS

### PATENT DOCUMENT

Patent Document 1: WO 2006/025583

### NON-PATENT DOCUMENTS

Non-Patent Document 1: International Journal of Pharmaceutics 293 (2005) 73-82
Non-Patent Document 2: Drug Design, Development and Therapy 11 (2017) 393-406
Non-Patent Document 3: Journal of Controlled Release 180 (2014) 10-24
Non-Patent Document 4: Journal of Controlled Release 206 (2015) 140-152
Non-Patent Document 5: Vaccine 29 (2011) 5393-5398
Non-Patent Document 6: Journal of Controlled Release 131 (2008) 14-18

### SUMMARY OF THE INVENTION

### PROBLEMS TO BE SOLVED BY THE INVENTION

As mentioned above, the conventional techniques for permeating hydrophilic drugs into the skin, that is, the technique of percutaneously absorbing involve the problems, so that a technique capable of avoiding use of a surfactant that inhibits percutaneous permeation of hydrophilic drugs, and can deliver hydrophilic drugs having a large molecular weight such as a protein from the skin into the body has been desired.

### MEANS TO SOLVE THE PROBLEMS

The present invention has been made in view of the above-mentioned problems, and is to provide a formulation containing a hydrophilic drug, an oily base, and optionally an oil-soluble percutaneous permeation enhancing substance by preparing a formulation (Solid in Oil Suspension; the S/O-S formulation) in which a hydrophilic drug is directly suspended in an oily base, and a method for percutaneously absorbing the hydrophilic drug. Here, when the oil-soluble percutaneous permeation enhancing substance is contained, provided is a method of percutaneously absorbing the hydrophilic drug, which does not substantially contain water.

That is, the present invention is as follows:
(1) A transdermal formulation containing a hydrophilic drug and an oily base, characterized in that at least a part of the hydrophilic drug exists in a suspended state with no dissolution in the oily base.
(2) The transdermal formulation described in the above-mentioned (1), which further comprises an oil-soluble percutaneous permeation enhancing substance.
(3) The transdermal formulation described in the above-mentioned (2), wherein the oil-soluble percutaneous permeation enhancing substance is at least one kind selected from the group consisting of phosphoglycerides, glycerides, fatty acid esters of sugar alcohols, fatty acid esters of glycols, fatty acids, terpenes and essential oils.
(4) The transdermal formulation described in the above-mentioned (2) or (3), wherein the oil-soluble percutaneous permeation enhancing substance is a terpene.
(5) The transdermal formulation described in the above-mentioned (2) or (3), wherein the oil-soluble percutaneous permeation enhancing substance is a fatty acid ester of glycerin.
(6) The transdermal formulation described in the above-mentioned (5), wherein the oil-soluble percutaneous permeation enhancing substance is an unsaturated fatty acid ester of glycerin.
(7) The transdermal formulation described in the above-mentioned (6), wherein a carbon chain of the unsaturated fatty acid is 16 or more and 22 or less.
(8) The transdermal formulation described in any one of the above-mentioned (2), (3) and (5) to (7), wherein the oil-soluble percutaneous permeation enhancing substance is glyceryl monooleate.
(9) The transdermal formulation described in any one of the above-mentioned (1) to (8), wherein a molecular weight of the hydrophilic drug is 500 Da to 200 kDa.
(10) The transdermal formulation described in any one of the above-mentioned (1) to (9), wherein a zeta potential of the hydrophilic drug is -50 to 10 mV.
(11) The transdermal formulation described in any one of the above-mentioned (1) to (10), which contains substantially no water.
(12) A method of improving percutaneous absorbability of a hydrophilic drug characterized in making, in a transdermal formulation containing a hydrophilic drug and an oily base, at least a part of the hydrophilic drug into a suspended state with no dissolution in the oily base.
(13) The method described in the above-mentioned (12), wherein the transdermal formulation further contains an oil-soluble percutaneous permeation enhancing substance.
(14) The method described in the above-mentioned (13), wherein the oil-soluble percutaneous permeation enhancing substance is at least one kind selected from the group consisting of phosphoglycerides, glycerides, fatty acid esters of sugar alcohols, fatty acid esters of glycols, fatty acids, terpenes and essential oils.
(15) The method described in the above-mentioned (13) or (14), wherein the oil-soluble percutaneous permeation enhancing substance is a terpene.
(16) The method described in the above-mentioned (13) or (14), wherein the oil-soluble percutaneous permeation enhancing substance is a monofatty acid ester of glycerin.
(17) The method described in the above-mentioned (16), wherein the oil-soluble percutaneous permeation enhancing substance is an unsaturated fatty acid ester of glycerin.
(18) The method described in the above-mentioned (17), wherein a carbon chain of the unsaturated fatty acid is 16 or more and 22 or less.
(19) The method described in any one of the above-mentioned (13), (14) and (16) to (18), wherein the oil-soluble percutaneous permeation enhancing substance is glyceryl monooleate.
(20) The method described in any one of the above-mentioned (12) to (19), wherein a molecular weight of the hydrophilic drug is 500 Da to 200 kDa.
(21) The method described in any one of the above-mentioned (12) to (20), wherein a zeta potential of the hydrophilic drug is -50 to 10 mV.
(22) The method described in any one of the above-mentioned (12) to (21), wherein the transdermal formulation contains substantially no water.
(23) A method for producing a percutaneous formulation, which comprises a step of mixing a hydrophilic drug and an oily base and a step of precipitating at least a part of the hydrophilic drug.

### EFFECTS OF THE INVENTION

In the transdermal formulation of the present invention, by making a formulation (S/O-S formulation) in which the powder of the hydrophilic drug is directly suspended in an oily base, use of surfactants which inhibit percutaneous permeation of the hydrophilic drug can be avoided and oil-soluble percutaneous permeation enhancing substances can be contained with a large amount, so that the hydrophilic drug such as proteins becomes to deliver through the skin into the body with several ten-times higher at the maximum than that of the aqueous solution.

### BRIEF DESCRIPTION OF THE DRAWINGS

Fig. 1 is a graph showing the results of a skin permeability test of a CMAP peptide in the S/O-S formulation, obtained in Example 1, containing a CMAP peptide and various kinds of oily bases.
Fig. 2 is a graph showing the results of a skin permeability test of OVA in the S/O-S formulation, obtained in Example 2, containing OVA, IPM and various kinds of oil-soluble percutaneous permeation enhancing substances.
Fig. 3 is a graph showing the relationship between the concentration of various kinds of oil-soluble percutaneous permeation enhancing substances and an amount of the OVA percutaneous permeation in the results of a skin permeability test of an S/O-S formulation obtained in Example 3 which contains OVA, IPM as well as ER290 (A), L195 (B), P90G (C) or MGOL-70 (D) as the oil-soluble percutaneous permeation enhancing substance.
Fig. 4 is a graph showing the results of a skin permeability test of OVA in the S/O-S formulation obtained in Example 4, containing OVA, IPM and various kinds of terpenes as oil-soluble percutaneous permeation enhancing substances.
Fig. 5 is a graph showing the relationship between the concentration of various kinds of oil-soluble percutaneous permeation enhancing substances and an amount of the OVA percutaneous permeation in the results of a skin permeability test of the S/O-S formulation obtained in Example 5, which contains OVA, IPM, and Nerolidol (A) or Geraniol (B) as the oil-soluble percutaneous permeation enhancing substance,.
Fig. 6 is a graph showing the results of a skin permeability test of OVA of an S/O-S formulation obtained in Example 7, containing two kinds of OVA particles having different particle sizes, IPM and MGOL-70.
Fig. 7 is a graph showing the results of a skin permeability test of OVA of the S/O-S formulation obtained in Example 8, containing OVA, MGOL-70 and various kinds of oily bases.
Fig. 8 is a graph showing the results of a hyaluronic acid skin permeability test of the S/O-S formulation obtained in Example 9, containing hyaluronic acid, MGOL-70 and squalane.
Fig. 9 is a fluorescence micrograph of a tissue section of porcine skin to which the S/O-S formulation obtained in Example 9, containing hyaluronic acid, MGOL-70 and squalene, was administered.

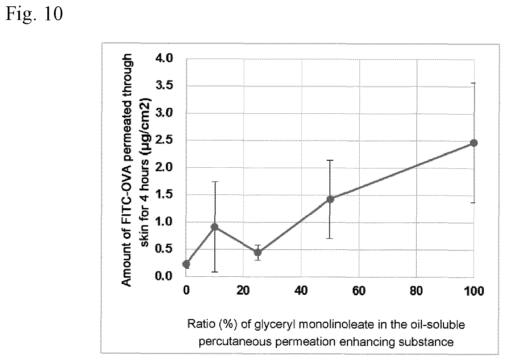

Fig. 10 is a graph showing the relationship between the addition ratio of glyceryl monolinoleate in the oil-soluble percutaneous permeation enhancing substance and an amount of the OVA percutaneously permeated in the results of a skin permeability test of the S/O-S formulation obtained in Example 11, which contains OVA, IPM and glyceryl monooleate and/or glyceryl monolinoleate as the oil-soluble percutaneous permeation enhancing substance.

### EMBODIMENT TO CARRY OUT THE INVENTION

### (Transdermal formulation)

The transdermal formulation of the present invention comprises a hydrophilic drug and an oily base, and at least a part of the hydrophilic drug is present in the oily base in a suspended state with no dissolution. The suspension in the present invention means a state in which solid particles are dispersed in a liquid, and in some cases, all or part of the solid particles may be precipitated. The transdermal formulation of the present invention is preferably in the state that the hydrophilic drug precipitated in the oily base is in a concentrated state at the contacting site with the skin. It can be considered that the concentrated hydrophilic drug is dissolved by a minute amount of water of the skin at the time of contacting with the skin and penetrates into the skin.

In the transdermal formulation of the present invention, an amount of the hydrophilic drug is not particularly limited as long as at least a part thereof can exist with no dissolution in the oily base and the drug can be added to the base in an optional ratio, and as the ratio (mass basis) of the drug to the oil-soluble base, it is 0.01:100 to 100:0.01, preferably 0.1:100 to 100:0.1, and more preferably 1:10 to 10:1. Incidentally, "at least a part of the hydrophilic drug" that exists in the oil-soluble base with no dissolution means that it is 10% by mass or more based on the total amount of the drug added to the base, preferably 50% by mass or more, more preferably 80% by mass or more, further preferably 90% by mass or more, and particularly preferably 99% by mass or more.

### (Hydrophilic drug)

The hydrophilic drug used in the present invention is not particularly limited as long as it is a solid at normal temperature (for example, see 25°C), at least a part of which is dissolved in water, and is an effective ingredient applicable to animals including humans in the fields of cosmetics and pharmaceuticals. Here, the drug at least a part of which is dissolved in water may be any material which corresponds to any of, for example, in a term indicating solubility defined in the general rules of Japanese Pharmacopeia, difficultly soluble (an amount of water required to dissolve 1 g of the drug is 100 mL or more and less than 1,000 mL), slightly difficultly soluble (an amount of water required to dissolve 1 g of the drug is 30 mL or more and less than 100 mL), slightly soluble (an amount of water required to dissolve 1 g of the drug is 10 mL or more and less than 30 mL), soluble (an amount of water required to dissolve 1 g of the drug is 1 mL or more and less than 10 mL), or extremely soluble (an amount of water required to dissolve 1 g of the drug is less than 1 mL).

A molecular weight of the hydrophilic drug used in the present invention is not particularly limited, and from the point that the transdermal formulation of the present invention can deliver the hydrophilic drug having a large molecular weight through the skin into the body, it is preferably the molecular weight of 500 Da or more, more preferably 1,000 Da (that is, 1 kDa) or less, and preferably 200 kDa or less, more preferably 100 kDa or less, and particularly preferably 50 kDa or less.

Examples of such a hydrophilic drug may include
polysaccharides such as hyaluronic acid and a salt thereof, heparin and a similar compound thereof, chitosan and the like;
proteins such as ovalbumin, casein, and collagen;
vitamins such as vitamin C and a derivative thereof, and retinol;
amino acids such as amino acid, tranexamic acid, and placental extract;
glycosides such as arbutin;
coenzymes such as coenzyme Q10;
proteinaceous formulations such as cholera toxin subunit B, epithelial growth factor, serum albumin, immunoglobulin, interleukin, interferon, human glucagon, human growth hormone, erythropoietin, platelet-derived growth factor, and insulin; and peptide formulations such as cancer vaccine, and allergy vaccine.

### (Oily base)

The oily base used in the present invention is not particularly limited as long as it is a liquid at normal temperature (for example, see 25°C), is substantially insoluble in water, has a viscosity, has a specific gravity lower than that of water, is used in the fields of cosmetics and pharmaceuticals, and does not exert any bad effects when it is contacted with the skin of animals including humans. Examples of such an oily base may include,
higher (polyvalent) alcohols such as cetanol, myristyl alcohol, oleyl alcohol, lauryl alcohol, cetostearyl alcohol, stearyl alcohol, behenyl alcohol, jojoba alcohol, chimyl alcohol, selachyl alcohol, batyl alcohol, hexyldecanol, isostearyl alcohol, 2-octyldodecanol, and dimer diol;
aralkyl alcohols and a derivative thereof such as benzyl alcohol and the like; long-chain fatty acids and a derivative thereof such as isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, dimer acid, and hydrogenated dimer acid;
hydrocarbons such as liquid paraffin (mineral oil), heavy liquid isoparaffin, light liquid isoparaffin, α-olefin oligomer, polyisobutene, hydrogenated polyisobutene, polybutene, squalane, olive-derived squalane, squalene, vaseline, and solid paraffin;
waxes such as candelilla wax, carnauba wax, rice wax, wood wax, beeswax, montan wax, ozokerite, ceresin, paraffin wax, microcrystalline wax, petrolatum, Fischer-Tropsch wax, polyethylene wax, and ethylene-propylene copolymer;
vegetable oils and fats such as coconut oil, palm oil, palm kernel oil, safflower oil, olive oil, castor oil, avocado oil, sesame oil, tea oil, evening primrose oil, wheat germ oil, perilla oil, apricot oil, almond oil, macadamia nut oil, hazelnut oil, kukui nut oil, rosehip oil, meadowfoam oil, persic oil, tea tree oil, peppermint oil, corn oil, rapeseed oil, sunflower oil, wheat germ oil, linseed oil, cotton seed oil, soybean oil, peanut oil, rice bran oil, cacao butter, shea butter, hydrogenated coconut oil, hydrogenated castor oil, jojoba oil, and hydrogenated jojoba oil;
animal oils and fats such as beef tallow, milk fat, horse fat, egg yolk oil, mink oil, and turtle oil;
animal waxes such as whale wax, lanolin, and orange roughy oil;
lanolins such as liquid lanolin, reduced lanolin, adsorption refined lanolin, lanolin acetate, liquid lanolin acetate, hydroxy lanolin, polyoxyethylene lanolin, lanolin fatty acid, hard lanolin fatty acid, lanolin alcohol, acetylated lanolin alcohol, (cetyl lanolyl) acetic acid ester and the like; sterols such as cholesterol, dihydrocholesterol, lanosterol, dihydrolanosterol, phytosterol, and cholic acid;
sapogenins;
saponins;
sterol esters such as cholesteryl acetate, cholesteryl nonanoate, cholesteryl stearate, cholesteryl isostearate, cholesteryl oleate, di(cholesteryl/behenyl/octyl dodecyl) N-lauroyl-L-glutamate, di(cholesteryl/octyl dodecyl) N-lauroyl-L-glutamate, di(phytosteryl/behenyl/octyl dodecyl) N-lauroyl-L-glutamate, di(phytosteryl/octyl dodecyl) N-lauroyl-L-glutamate, cholesteryl 12-hydroxystearate, cholesteryl macademiate, phytosteryl macademiate, phytosteryl isostearate, soft cholesteryl lanolate, hard cholesteryl lanolate, long-chain branched fatty acid cholesterol ester, and long-chain α-hydroxy fatty acid cholesterol ester;
acylsarcosine alkyl esters such as N-lauroylsarcosine isopropyl;
lipid complexes such as phospholipid-cholesterol complex, and phospholipid-phytosterol complex;
long-chain fatty acid esters such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, lanolin fatty acid octyl dodecyl , hexyldecyl dimethyloctanoate, octyldodecyl erucate, hardened castor oil isostearate, ethyl oleate, avocado oil fatty acid ethyl ester, isopropyl myristate , isopropyl palmitate, octyl palmitate, isopropyl isostearate, lanolin fatty acid isopropyl ester and the like; dicarboxylic acid esters such as diethyl sebacate, diisopropyl sebacate, dioctyl sebacate, diisopropyl adipate, dibutyloctyl sebacate, diisobutyl adipate, dioctyl succinate, and triethyl citrate;
oxyacid esters such as cetyl lactate, diisostearyl malate, hydrogenated castor oil and monoisostearate;
polyvalent alcohol fatty acid esters such as glyceryl trioctanoate (glyceryl tri-2-ethylhexanoate), glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate/eicosadioate, trimethylol propane trioctanoate, trimethylol propane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, pentaerythrityl hydrogenated rosinate, ditrimethylolpropane triethylhexanoate, ditrimethylolpropane (isostearate/sebacate), pentaerythrityl triethylhexanoate, (hydroxystearate/stearate/rosinate) dipentaerythrityl , diglyceryl diisostearate, polyglyceryl tetraisostearate, polyglyceryl-10 nonaisostearate, polyglyceryl-8 deca(erucate/isostearate/ricinoleate), diglyceryl oligo ester (hexyldecanoate/sebacate), glycol distearate (ethylene glycol distearate), 3-methyl-1,5-pentanediol dineopentanoate, 2,4-diethyl-1,5-pentanediol dineopentanoate and the like; derivatives of dimer acids or dimer diols such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, diisostearate dimer dilinoleyl, dimer dilinoleyl hydrogenated rosin condensate, hydrogenated castor oil dimer dilinoleate, and hydroxyalkyl dimer dilinoleyl ether;
fatty acid alkanol amides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (partamide MEA), palmitic acid diethanolamide (partamide DEA), and coconut oil fatty acid methyl ethanolamide (cocamidemethyl MEA);
silicones such as dimethicone (dimethylpolysiloxane), highly polymerized dimethicone (highly polymerized dimethylpolysiloxane), cyclomethicone (cyclic dimethylsiloxane) such as decamethylcyclopentasiloxane (simply also referred to as cyclopentasiloxane), phenyltrimethicone, diphenyldimethicone, phenyldimethicone, stearoxypropyldimethylamine, (aminoethylaminopropylmethicone/dimethicone) copolymer, dimethiconol, dimethiconol crosspolymer, silicone resin, silicone rubber, amino-modified silicones such as aminopropyldimethicone and amodimethicone, cation-modified silicones, polyether-modified silicones such as dimethicone copolyol and the like, polyglycerin-modified silicones, sugar-modified silicones, carboxylic acid-modified silicones, phosphate-modified silicones, sulfate-modified silicones, alkyl-modified silicones, fatty acids-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymer; and
fluorine-based oily bases such as perfluorodecane, perfluorooctane, and perfluoropolyether.

Among these, preferable are long-chain fatty acids such as oleic acid; vegetable oils and fats such as olive oil and jojoba oil; esters of alkyl(s) having 1 to 8 carbon atoms and fatty acid(s) having 12 to 18 carbon atoms such as isopropyl myristate, isopropyl palmitate, octyl palmitate and isopropyl isostearate; and hydrocarbons such as squalane, and more preferable are isopropyl myristate and squalane.

### (Oil-soluble percutaneous permeation enhancing substance)

An oil-soluble percutaneous permeation enhancing substances may be added to the transdermal formulation of the present invention. The oil-soluble percutaneous permeation enhancing substance is not particularly limited as long as the substance is soluble in an oily base, has a function of enhancing permeation of a hydrophilic drug into the skin when the drug comes into contact with the skin, and is a component applicable to animals including human in the fields of cosmetics and pharmaceuticals. An amount of the oil-soluble percutaneous permeation enhancing substance is not particularly limited, and can be incorporated into the base at an optional ratio. A ratio (volume standard) of the added oil-soluble percutaneous permeation enhancing substance to the oily base is 0.01:100 to 100:0.01, preferably 0.1:100 to 100:0.1, more preferably 1:10 to 10:1, and particularly preferably 1:2 to 2:1. In the transdermal formulation of the present invention, it is preferable to substantially contain no water. "Substantially contain no water" means that a weight ratio of water to the transdermal formulation is less than 10%, preferably less than 5%, further preferably less than 2%, and most preferably less than 1%. If water is contained, the transdermal formulation can be separated into an aqueous phase and an oil phase, leading to exerting adverse effect on the percutaneous absorbability. In particular, in the embodiment containing the oil-soluble percutaneous permeation enhancing substance, a weight ratio of water to the oil-soluble percutaneous permeation enhancing substance is, for example, less than 20%, preferably less than 10%, more preferably less than 5%, further preferably less than 3%, and most preferably less than 1%. If the amount of water in the oil-soluble percutaneous permeation enhancing substance is large, gelation may occur in the transdermal formulation, so that it could lead to exerting adverse effect on the percutaneous absorbability.

Examples of the oil-soluble percutaneous permeation enhancing substances may include phosphoglycerides, glycerides, fatty acid esters of sugar alcohols, fatty acid esters of glycols, fatty acids, terpenes, essential oils and the like.

Examples of the phosphoglycerides may include lecithin (phosphatidylcholine).

The glycerides (i.e., fatty acid ester of glycerin) may be either a saturated fatty acid ester or unsaturated fatty acid ester of glycerin, preferably an unsaturated fatty acid ester of glycerin, more preferably an ester of unsaturated fatty acid(s) having 16 to 22 carbon atoms and glycerin, for example, glyceryl monooleate, glyceryl dioleate, glyceryl monolinoleate and glyceryl monolinolenate, and particularly preferably glyceryl monooleate, glyceryl monolinoleate or a combination thereof.

Examples of the fatty acid esters of sugar alcohols may include sucrose oleic acid esters and sorbitan oleate, and examples of the fatty acid esters of glycols may include propylene glycol oleate and polyethylene glycol oleate.

Examples of the fatty acids may include oleic acid, linoleic acid, linolenic acid and the like, and examples of the terpenes may include farnesol, β-citronellol, citral, geraniol, nerolidol and the like.

The oil-soluble percutaneous permeation enhancing substance is preferably lecithin, terpene, glyceryl monooleate, glyceryl monolinoleate or a combination thereof.

### (Preparation of transdermal formulation)

The method for preparing a transdermal formulation in the present invention includes (i) a step of mixing an oily base and a hydrophilic drug. The transdermal formulation in the present invention is required to present at least a part of the hydrophilic drug in a suspended state in an oily base without being dissolved, and preferably precipitated. For this reason, it is preferable to include (ii) a step of pulverizing the hydrophilic drug. The step (ii) can be carried out before and/or after the step (i), and the pulverizing method which can be used in the step (ii) may be mentioned a well-known method, for example, a homogenizer-crushing method, an ultrasonic crushing method, a bead pulverization method, a mortar pulverization method, a spray drying method and the like.

The step (ii) before the step (i) may be carried out by, for example, pulverizing and atomizing the hydrophilic drug by the above-mentioned pulverization method, and may be purchased the hydrophilic drug previously pulverized to a predetermined particle size. On the other hand, the step (ii) after the step (i) may be carried out by pulverizing and atomizing the hydrophilic drug in the oily base by the above-mentioned pulverization method to suspend in the oily base. Also, before the step (i), the step (ii) is carried out to pulverize and atomize the hydrophilic drug, further after the step (i), the step (ii) is additionally carried out to pulverize and atomize the hydrophilic drug. The pulverization conditions are not particularly limited as long as the drug molecule is not damaged by the pulverization. For example, when a homogenizer is used, its rotation speed is preferably 15,000 rpm to 20,000 rpm.

The smaller the particle size of the hydrophilic drug in the present invention is, the higher the transdermal effect is, and the particle size is preferably 100 µm or less, more preferably 50 µm or less, most preferably 10 µm or less, and preferably 0.01 µm or more, more preferably 0.1 µm or more, and most preferably 0.3 µm or more.

For example, when measured by an electrophoretic light scattering method in phosphate buffered physiological saline (PBS) at 25°C according to the measurement method described in Example 6 described later, a zeta potential (ζ potential) of the hydrophilic drug in the present invention is, preferably -50 to 10 mV, more preferably - 40 to 0 mV, and particularly preferably a negative value, for example, -30 to -0.001 mV.

Further, the method for preparing a transdermal formulation in the present invention may comprise (iii) a step of mixing an oil-soluble percutaneous permeation enhancing substance. The step (iii) may be carried out in an optional order in the preparation method comprising the step (i). For example, before the above-mentioned mixing step (i), it may be carried out by (iii-a) mixing the oily base and the oil-soluble percutaneous permeation enhancing substances, or (iii-b) mixing the hydrophilic drug and the oil-soluble percutaneous permeation enhancing substances. Alternatively, after the above-mentioned step (i), it may be carried out by (iii-c) mixing a mixture of the oily base and the hydrophilic drug, and the oil-soluble percutaneous permeation enhancing substances. At that time, the above-mentioned step (ii) may be carried out before and/or after the step (i), and may be carried out before and/or after the step (iii-b), or before and/or after the step (iii-c). That is, the method for preparing a transdermal formulation in the present invention typically comprises the step (i), preferably comprises the steps (i) and (ii), more preferably comprises the steps (i), (ii) and (iii), and depending on the each component and the like contained in the formulation, it may be carried out in the order of the step (i) → the step (ii), in the order of the step (ii) → the step (i) → the step (ii), in the order of the step (i) → the step (ii) → the step (iii-c), in the order of the step (iii-a) → the step (i) → the step (ii), in the order of the step (iii-b) → the step (i) → the step (ii), or in the order of the step (iii-b) → the step (ii) → the step (i) and the like.

The transdermal formulation in the present invention thus obtained may be used without further preparation, or may be used in the form of an ointment, a cream, a lotion, a spray or a patch by further subjecting to preparation by a method known to those skilled in the art.

The present invention is also directed to a method for improving percutaneous absorbability of a hydrophilic drug characterized in making, in a transdermal formulation containing a hydrophilic drug and an oily base, at least a part of the hydrophilic drug in the oily base into a suspended state without no dissolution. In such a method, the meanings and preferred embodiments of terms such as "hydrophilic drug", "oily base", "transdermal formulation" and the like are as mentioned above. In addition, in the present invention, "percutaneous absorbability is improved" means, for example, that, when the formulation of the present invention, and a PBS solution and an S/O formulation are compared to each other in an in vitro skin permeability test as described in Example 9 mentioned later, the former shows a higher value than the latter in an amount of skin permeation.

The present invention also relates to a method for percutaneously administering a hydrophilic drug, comprising a step of bringing the formulation into contact with a skin, characterized in that the formulation is a transdermal formulation containing the hydrophilic drug and an oily base, and at least a part of the hydrophilic drug exists in a suspended state with no dissolution in the oily base. In such a method, the meanings and preferred embodiments of terms such as "hydrophilic drug", "oily base", "transdermal formulation" and the like are as mentioned above. In addition, in the present invention, "a step of bringing the formulation into contact with the skin" is, for example, carried out by optionally coating, spraying or pasting the formulation in the form of an ointment, a cream, a lotion, a spray or a patch to an appropriate portion on surface of human skin, depending on the preparation of the formulation.

Hereinafter, Examples of the present invention are shown, but the present invention is not limited thereto.

### EXAMPLES

### (Example 1: Effect of oily base on skin permeability of milk allergy peptide (cow's milk allergy peptide, CMAP) vaccine)

### <Preparation of Formulation>

In a 5 mL-volume glass vial bottle was weighed 8 mg of a CMAP peptide (sequence: LLDAQSAPLRVYVEELKP) labelled with FITC (Fluorescein isothiocyanate), and after adding 4 mL of isopropyl myristate (IPM; available from Tokyo Chemical Industry Co., Ltd.) thereto, the mixture was stirred, by using a polytron homogenizer PT2500 (manufactured by Kinematika) equipped with a 7 mm diameter shaft, at 20,000 rpm for 2 minutes to obtain a Solid in Oil Suspension(S/O-S) formulation containing 2 mg/mL of peptide particles.

The 2 mg/mL S/O-S formulation was mixed with each of IPM, liquid paraffin (available from Wako Pure Chemical Industries, Ltd.), volatile silicone (KF-995; available from Shin-Etsu Chemical Co., Ltd.), olive oil (available from Wako Pure Chemical Industries, Ltd.), jojoba oil (available from Wako Pure Chemical Industries, Ltd.), oleic acid (available from Wako Pure Chemical Industries, Ltd.) and linoleic acid (available from Wako Pure Chemical Industries, Ltd.) at 1:1 (v/v) to obtain S/O-S formulations of 1 mg/mL CMAP containing various kinds of the oily bases.

### <In vitro skin permeability test>

Yucatan micropig (YMP) skin purchased from Charles River was set in a Franz-type diffusion cell (effective area 0.785 cm²), and its receiver phase was filled with 5 mL of PBS solution. To its donor phase was added 200 µL of the S/O-S preparation and it was incubated at 32°C. After 24 hours, the YMP skin was recovered, washed with ethanol and pure water (Milli-Q water), finely chopped, and shaken at high speed in a PBS solution for 24 hours. The amount of the FITC-labeled CMAP peptide extracted in the PBS solution was quantified by using a fluorescent plate reader.

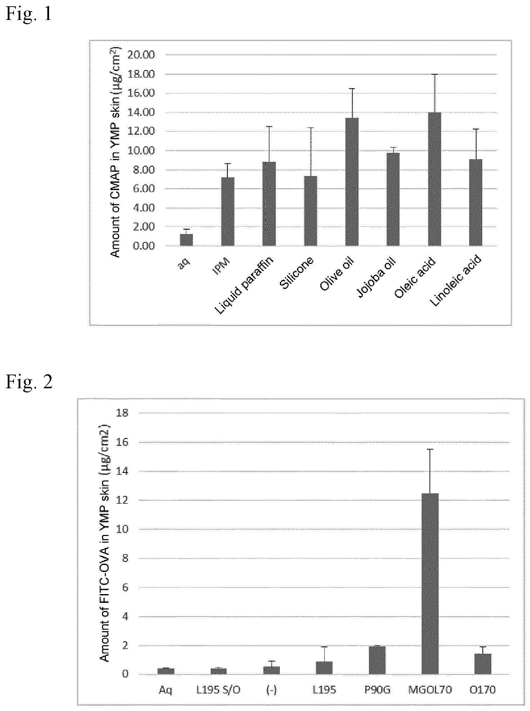

Figure 1 shows the amount of skin permeation of the CMAP peptide when the S/O-S formulations with various oily bases were used.

It was found that when olive oil and oleic acid were used as the oily bases, a high enhancing effect of percutaneous permeation could be exhibited.

### (Example 2: Effect of oil-soluble percutaneous permeation enhancing substance on skin permeability of ovalbumin (OVA))

### <Preparation of Formulation>

In a 5 mL-volume glass vial bottle was weighed 8 mg of OVA (available from Sigma-Aldlich) labeled with FITC (Fluorescein isothiocyanate), and after adding 4 mL of IPM thereto, the mixture was stirred by using a polytron homogenizer PT2500 equipped with a 7 mm diameter shaft, at 20,000 rpm for 2 minutes to obtain an S/O-S formulation containing 2 mg/mL OVA particles.

Sucrose lauric acid ester (L195; available from Mitsubishi-Chemical Foods Corporation), soybean-derived lecithin Phospholipon 90G (P90G; available from H. Holstein Co., Ltd.), glyceryl monooleate (MGOL-70; available from Nikko Chemicals Co., Ltd.) or sucrose oleic acid ester (0170; available from Mitsubishi-Chemical Foods Corporation) was each dissolved in IPM so that it became 50 mg/mL, and these were each mixed with the above-mentioned S/O-S formulation at 1:1 (v/v) to obtain S/O-S formulations containing various kinds of oil-soluble percutaneous permeation enhancing substances with 1 mg/mL OVA.

### <In vitro skin permeability test>

Yucatan micropig (YMP) skin purchased from Charles River skin was set in a Franz-type diffusion cell (effective area 0.785 cm²), and its receiver phase was filled with 5 mL of a PBS solution. To its donor phase was added 200 µL of the S/O-S preparation and it was incubated at 32°C. After 24 hours, the YMP skin was recovered, washed with ethanol and Milli-Q water, finely chopped and shaken at high speed in a PBS solution for 24 hours. The amount of the FITC-labeled OVA extracted with the PBS solution was quantified by using a fluorescent plate reader. Also, as controls, a PBS solution (OVA concentration 1 mg/mL), an S/O-S formulation (OVA concentration 1 mg/mL) containing no oil-soluble percutaneous permeation enhancing substance, and S/O nano particles (OVA concentration 1 mg/mL) prepared by using L195 were used.

Figure 2 shows the enhancing effect of percutaneous permeation by various oil-soluble percutaneous permeation enhancing substances on the S/O-S formulation.

By adding MGOL-70 to the S/O-S formulation, a high enhancing effect of percutaneous permeation was exhibited, which was 15-times higher than that of the case without the addition and 30-times higher than that of the PBS solution.

### (Example 3: Relationship between skin permeability of ovalbumin (OVA) and concentration of oil-soluble percutaneous permeation enhancing substance)

### <Preparation of Formulation>

In a 5 mL-volume glass vial bottle was weighed 8 mg of OVA labeled with FITC (Fluorescein isothiocyanate), and after adding 4 mL of IPM thereto, the mixture was stirred, by using a polytron homogenizer PT2500 (manufactured by Kinematika) equipped with a 7 mm diameter shaft, at 20,000 rpm for 2 minutes to obtain an S/O-S formulation containing 2 mg/mL of OVA particles.

Sucrose lauric acid ester (L-195), sucrose erucic acid ester (ER-290; available from Mitsubishi-Chemical Foods Corporation), soybean-derived lecithin Phospholipon 90G (P90G) and glyceryl monooleate (MGOL-70) was each dissolved in IPM to have a concentration of 2, 10, 50 or 100 mg/mL, and these were each mixed with the above-mentioned S/O-S formulation at 1:1 (v/v) to obtain S/O-S formulations containing various oil-soluble percutaneous permeation enhancing substances with 1 mg/mL OVA.

### <In vitro skin permeability test>

Yucatan micropig (YMP) skin purchased from Charles River was set in a Franz-type diffusion cell (effective area 0.785 cm²), and its receiver phase was filled with 5 mL of a PBS solution. To its donor phase was added 200 µL of the S/O-S preparation and it was incubated at 32°C. After 24 hours, the YMP skin was recovered, washed with ethanol and Milli-Q water, finely chopped and shaken at high speed in a PBS solution for 24 hours. The amount of the FITC-labeled OVA extracted in the PBS solution was quantified by using a fluorescent plate reader.

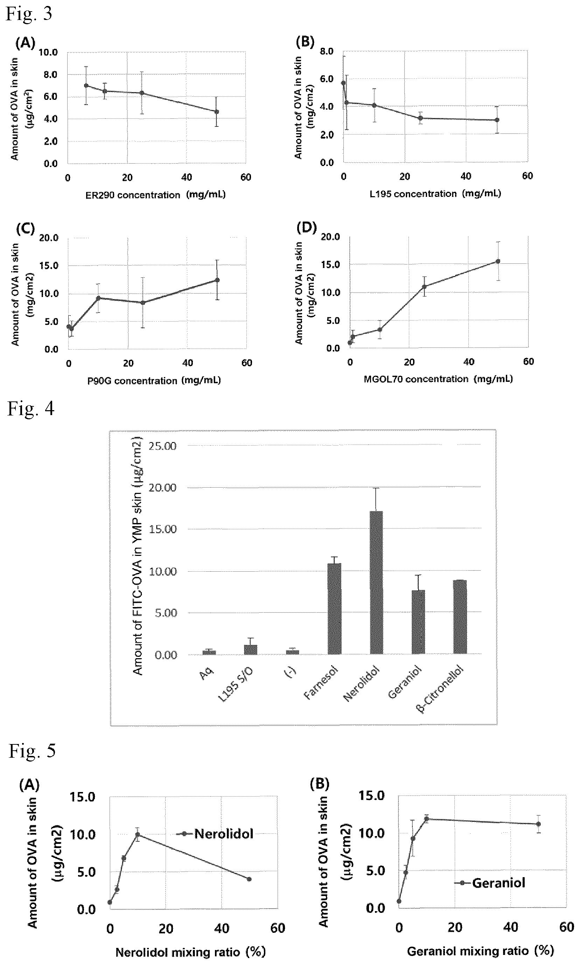

Figure 3 shows the relationship between the concentration of various oil-soluble percutaneous permeation enhancing substances in the S/O-S formulation and the enhancing effect of percutaneous permeation.

As the oil-soluble percutaneous permeation enhancing substances, ER290 (A), L195 (B), P90G (C) and MGOL-70 (D) were used. It was found that, ER290 and L195 tend to inhibit percutaneous permeation of OVA, while P90G and MGOL-70 tend to enhance it.

### (Example 4: Effect of terpenes on skin permeability of ovalbumin (OVA))

### <Preparation of Formulation>

In a 5 mL-volume glass vial bottle was weighed 8 mg of OVA labeled with FITC (Fluorescein isothiocyanate), and after adding 4 mL of IPM containing 2 mg/mL of Phospholipon 90G thereto, the mixture was stirred, by using a polytron homogenizer PT2500 (manufactured by Kinematika) equipped with a 7 mm diameter shaft, at 20,000 rpm for 2 minutes to obtain an S/O-S formulation containing 2 mg/mL of OVA particles.

A mixture of 100 µL of Farnesol, Nerolidol, Geraniol or β-Citronellol and 900 µL of IPM was each mixed with the above-mentioned S/O-S formulation at 1:1 (v/v) to obtain 1 mg/mL of S/O-S formulations containing 5 vol% of terpene.

### <In vitro skin permeability test>

Yucatan micropig (YMP) skin purchased from Charles River was set in a Franz-type diffusion cell (effective area 0.785 cm²), and its receiver phase was filled with 5 mL of a PBS solution. To its donor phase was added 200 µL of the S/O-S preparation and it was incubated at 32°C. After 24 hours, the YMP skin was recovered, washed with ethanol and Milli-Q water, finely chopped and shaken at high speed in a PBS solution for 24 hours. The amount of the FITC-labeled OVA extracted in the PBS solution was quantified by using a fluorescent plate reader. Also, as a control, a PBS solution (OVA concentration 1 mg/mL), an S/O-S formulation containing no emulsifier (OVA concentration 1 mg/mL), and S/O nano particles (OVA concentration 1 mg/mL) prepared by using L195 were used.

Figure 4 shows the enhancing effect of percutaneous permeation by terpenes on the S/O-S formulation. From the results, it was clarified that the S/O-S formulation containing Nerolidol showed a high enhancing effect of percutaneous permeation, which was 36-times higher than the PBS solution.

### (Example 5: Relationship between skin permeability of ovalbumin (OVA) and terpene concentration)

### <Preparation of Formulation>

In a 5 mL-volume glass vial bottle was weighed 8 mg of OVA labeled with FITC (Fluorescein isothiocyanate), and after adding 4 mL of IPM containing 2 mg/mL of Phospholipon 90G thereto, the mixture was stirred, by using a polytron homogenizer PT2500 (manufactured by Kinematika) equipped with a 7 mm diameter shaft, at 20,000 rpm for 2 minutes to obtain an S/O-S formulation containing 2 mg/mL OVA particles.

To a 5 mL-volume vial was added 50, 100, 200 or 1,000 µL of Nerolidol or Geraniol, and IPM was further added to adjust the total volume to 1,000 µL. The obtained mixture of terpene and IPM was further mixed with the above-mentioned S/O-S formulation at 1:1 (v/v) to obtain 1 mg/mL of S/O-S formulations containing 2.5, 5, 10 or 50 vol% terpene.

### <In vitro skin permeability test>

Yucatan micropig (YMP) skin purchased from Charles River was set in a Franz-type diffusion cell (effective area 0.785 cm²), and its receiver phase was filled with 5 mL of a PBS solution. To its donor phase was added 200 µL of the S/O-S preparation and it was incubated at 32°C. After 24 hours, the YMP skin was recovered, washed with ethanol and Milli-Q water, finely chopped and shaken at high speed in a PBS solution for 24 hours. The amount of the FITC-labeled OVA extracted in the PBS solution was quantified using a fluorescent plate reader.

As the oil-soluble percutaneous permeation enhancing substances, Nerolidol (A) and Geraniol (B) were used. Figure 5 shows the relationship between the concentration of the terpenes added to the S/O-S formulation and the enhancing effect of percutaneous permeation. Nerolidol exhibited a maximum effect of percutaneous permeation at the addition amount of 5-10 vol%, and when it was added excessively, its enhancing effect of permeation was decreased, whereas Geraniol did not decrease the enhancing effect of percutaneous permeation even when the added amount was increased up to 50%.

### (Example 6: Effect of percutaneous permeation enhancement on hydrophilic polymer of S/O-S formulation containing MGOL-70)

### <Preparation of Formulation>

In a 5 mL-volume glass vial bottle was weighed 8 mg of various kinds of hydrophilic polymers shown in the following Table 1, and after adding 4 mL of IPM thereto, the mixture was stirred, by using a polytron homogenizer PT2500 (manufactured by Kinematika) equipped with a 7 mm diameter shaft, at 20,000 rpm for 2 minutes to obtain S/O-S formulations each containing 2 mg/mL various hydrophilic polymers.

MGOL-70 was dissolved in IPM to have a concentration of 50 mg/mL, and mixed with the above-mentioned S/O-S formulation at 1:1 (v/v) to obtain 1 mg/mL hydrophilic polymer S/O-S formulation containing 25 mg/mL MGOL-70.

### <In vitro skin permeability test>

Yucatan micropig (YMP) skin purchased from Charles River was set in a Franz-type diffusion cell (effective area 0.785 cm²), and its receiver phase was filled with 5 mL of a PBS solution. To its donor phase was added 200 µL of the S/O-S preparation and it was incubated at 32°C. After 24 hours, the YMP skin was recovered, washed with ethanol and Milli-Q water, finely chopped and shaken at high speed in a PBS solution for 24 hours.

The amount of the FITC-labeled hydrophilic polymer extracted in the PBS solution was quantified by using a fluorescent plate reader.

Green fluorescent protein (GFP) was quantified based on the fluorescence intensity derived from the protein. Horseradish peroxidase (HRP) was quantified by the absorbance at 450 nm after subjecting to a coloring reaction with a 3,3',5,5'-tetramethylbenzidine (TMB) solution.

From Table 1, it was shown that the S/O-S formulation containing MGOL-70 enhanced percutaneous permeation of various kinds of hydrophilic polymers. Further, the behaviors of GFP or HRP suggested the possibility that the protein could be permeated into the skin without being denatured.

**[Table 1]**

| Hydrophilic polymer | Molecular weight | pl | Skin permeation amount of PBS solution (*µ*g/cm²) | Skin permeation amount of S/O-S preparation (*µ*g/cm²) | Enhancement effect (fold) | Drug determination method in skin |
|---|---|---|---|---|---|---|
| Ovalubumin | 45 kDa | 4.9 | g/cm²) 0.41 | 12.5 | 30 | FITC-label |
| Horseraddish peroxidase | 44 kDa | 3.0-9.0 | 0.98 | 53.3 | 54 | peroxidase activity |
| Bovin serum albumin | 66.5 kDa | 4.7 | 3.6 | 32.1 | 8.9 | FITC-label |
| Green fluorescent protein | 29 kDa | 6.1 | 4.2 | 35.0 | 8.4 | fluorescence intensity |
| Cholera toxin subunit B | 11.6∗5 kDa (pentamer) | 6.6 | 9.8 | 39.4 | 4.0 | FITC-label |
| Lysozyme | 14.3 kDa | 11 | 4.0 | 6.0 | 1.5 | FITC-label |
| Peptide (Crp A) | 1426 Da | 8.5 | 1.90 | 6.5 | 3.4 | N-terminal FITC-label |
| Peptide (CMAP, from Beta-lactoglobulin) | 2041 Da | 4.7 | 1.30 | 11.1 | 15 | N-terminal FITC-label |
| Oligo Hyaluronate | av. 8000 Da | | 1.30 | 15.8 | 12.6 | FAC-label |

### <Measurement of zeta potential>

In each 1 mL of a PBS solution was dissolved 1 mg of a part (7 kinds) of the various kinds of hydrophilic polymers shown in the above-mentioned Table 1, and a zeta potential was measured by an electrophoretic light scattering method at 25°C using Zetasizer Nano ZS (Malvern Panalytical Ltd.). The results are shown in Table 2.

**[Table 2]**

| Hydrophilic polymer | ζ potential (mV) | Skin permeation amount of S/O-S preparation (*µ*g/cm²) |
|---|---|---|
| Ovalubumin | -12.80 | 12.5 |
| Horseraddish peroxidase | 0.007 | 53.3 |
| Bovin serum albumin | -16.7 | 32.1 |
| Green fluorescent protein | -9.52 | 35.0 |
| Cholera toxin subunit B | -3.29 | 39.4 |
| Lysozyme | 8.84 | 6.0 |
| Peptide (Crp A) | - | 6.5 |
| Peptide (CMAP, from Beta-lactoslobutin) | -21.2 | 11.1 |
| Oligo Hyaluronate | - | 15.8 |

### (Example 7: Relationship between particle size and permeation amount)

### <Preparation of Formulation>

In a 5 mL-volume glass vial bottle was weighed 8 mg of OVA labeled with FITC (Fluorescein isothiocyanate), and after adding 4 mL of IPM thereto, the mixture was stirred, by using a polytron homogenizer PT2500 (manufactured by Kinematika) equipped with a 7 mm diameter shaft, at 20,000 rpm for 2 minutes (S/O-S-2) to obtain an S/O-S formulation containing 2 mg/mL OVA particles. The obtained S/O-S formulation was sieved through a 635 mesh (20 µm) and classified according to the size of the particles. MGOL-70 was added thereto to have a concentration of 25 mg/mL, and the particle content was adjusted to 1 mg/mL to obtain an S/O-S formulation 1 (20 µm or more) and an S/O-S preparation 2 (20 µm or less).

### <In vitro skin permeability test>

Yucatan micropig (YMP) skin purchased from Charles River was set in a Franz-type diffusion cell (effective area 0.785 cm²), and its receiver phase was filled with 5 mL of PBS solution. To its donor phase was added 200 µL of the S/O-S preparation and it was incubated at 32°C. After 24 hours, the YMP skin was recovered, washed with ethanol and pure water (Milli-Q water), finely chopped and shaken at high speed in a PBS solution for 24 hours. The amount of the FITC-labeled OVA extracted in the PBS solution was quantified by using a fluorescent plate reader.

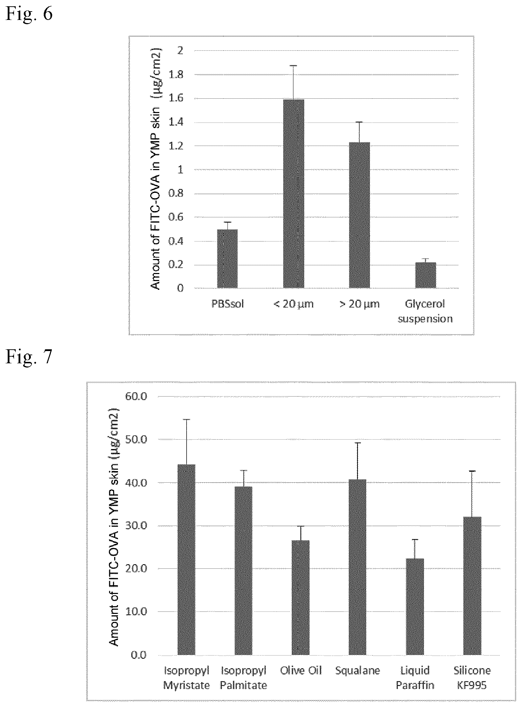

Figure 6 shows the results of the skin permeability test. It suggested the possibility that the smaller the particle size was, the higher the permeability into the skin was.

### (Example 8: Investigation of kind of base oil)

### <Preparation of Formulation>

In a 5 mL-volume glass vial bottle was weighed 4 mg of OVA labeled with FITC (Fluorescein isothiocyanate), and each 4 mL of an oily base (isopropyl myristate, isopropyl palmitate, olive oil, squalane, liquid paraffin or volatile silicone KF-995) containing 25 mg/mL MGOL-70 was added thereto. The mixture was stirred, by using a polytron homogenizer PT2500 (manufactured by Kinematika) equipped with a 7 mm diameter shaft at 20,000 rpm, for 2 minutes to obtain S/O-S formulations containing 1 mg/mL OVA particles.

### <In vitro skin permeability test>

Yucatan micropig (YMP) skin purchased from Charles River was set in a Franz-type diffusion cell (effective area 0.785 cm²), and its receiver phase was filled with 5 mL of PBS solution. To its donor phase was added 200 µL of the S/O-S preparation and it was incubated at 32°C. After 24 hours, the YMP skin was recovered, washed with ethanol and pure water (Milli-Q water), finely chopped and shaken at high speed in a PBS solution for 24 hours. The amount of the FITC-labeled OVA extracted in the PBS solution was quantified by using a fluorescent plate reader.

Table 3 and Figure 7 show the results of the skin permeability test. A high skin permeation effect was exhibited by using an oily base such as isopropyl myristate, isopropyl palmitate, and squalane.

**[Table 3]**

| | Isopropyl myristate | Isopropyl palmitate | Olive oil | Squalane | Liquid paraffine | Silicone KF-995 |
|---|---|---|---|---|---|---|
| Average skin permeation amount (*µ*g/cm²) | 44.3 | 39.1 | 26.6 | 40.8 | 22.3 | 32.0 |
| SD | 10.4 | 3.6 | 3.2 | 8.5 | 4.5 | 10.7 |

### (Example 9: Percutaneous permeation of oligo hyaluronic acid)

### <Preparation of Formulation>

In a 5 mL-volume glass vial bottle was weighed 4 mg of oligo hyaluronic acid (available from Kewpie Corporation) labeled with FITC (Fluorescein isothiocyanate), 4 mL of squalane containing 25 mg/mL of MGOL-70 was added thereto. The mixture was stirred by using a polytron homogenizer PT2500 (manufactured by Kinematika) equipped with a 7 mm diameter shaft, at 20,000 rpm for 2 minutes to obtain an S/O-S formulation containing 1 mg/mL FITC-labeled oligo hyaluronic acid.

On the other hand, 1 mg FITC-labeled oligo hyaluronic acid dissolved in pure water and 20 mg sucrose erucic acid ester ER-290 dissolved in cyclohexane were added to a glass vial. The oil phase and the aqueous phase were stirred, by using a polytron homogenizer PT2500 (manufactured by Kinematika) equipped with a 12 mm diameter shaft, at 26,000 rpm for 2 minutes to prepare a Water-in-oil (W/O) emulsion. This W/O emulsion was lyophilized and the obtained paste state surfactant-drug complex was dispersed in 1 mL of IPM to obtain an S/O formulation containing 1 mg/mL of FITC-labeled oligo hyaluronic acid, which was used as a control.

Further, 1 mg FITC-labeled oligo hyaluronic acid was dissolved in 1 mL of PBS solution, which was used as a PBS solution containing 1 mg/mL FITC-labeled oligo hyaluronic acid.

### <In vitro skin permeability test>

The subcutaneous tissue of SPF pig skin (produced by JA) purchased from KAC Co., Ltd. was removed, and set in a Franz-type diffusion cell (effective area 0.785 cm²), and its receiver phase was filled with 5 mL of PBS solution. To its donor phase were added 200 µL of an S/O-S preparation, an S/O preparation and a PBS solution, respectively, and it was incubated at 32°C. After 24 hours, the pig skin was recovered, washed with ethanol and pure water (Milli-Q water), finely chopped and shaken at high speed in a PBS solution for 24 hours. The amount of the FITC-labeled oligo hyaluronic acid extracted in the PBS solution was quantified by using a fluorescent plate reader.

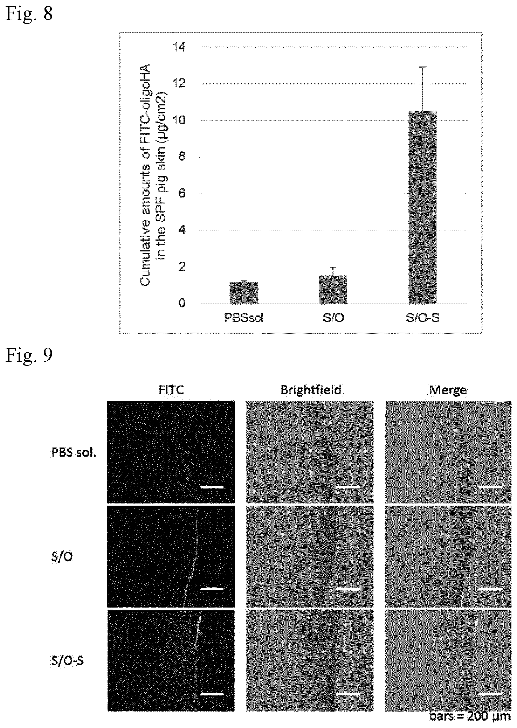

Table 4 and Figure 8 show the results of the skin permeability test. In the case of the S/O-S formulation, oligo hyaluronic acid could be percutaneously permeated with a 9 times higher efficiency than the PBS solution.

**[Table 4]**

| | Average amount (*µ*g/cm²) | Standard deviation |
|---|---|---|
| PBSsol | 1.17 | 0.07 |
| S/0 | 1.54 | 0.44 |
| S/0-S | 10.52 | 2.40 |

### <Fluorescence microscope observation>

In the same manner as the in vitro percutaneous permeation test, each of the S/O-S formulation, the S/O formulation and the PBS solution of the FITC-labeled oligo hyaluronic acid was administered to edible pig skin, and after 24 hours, the skin was washed with ethanol and Milli-Q water.

The washed skin was instantly frozen in an OCT compound at -80°C and 15 µm of tissue section was prepared by using a cryostat microtome.

The obtained skin section was observed by using a fluorescence microscope manufactured by Keyence. Figure 9 shows the results.

In the skin to which the S/O-S formulation was administered, green fluorescence was observed also from the deep part of the skin, and it was suggested that the oligo hyaluronic acid was permeated into inside the skin.

### (Example 10: Percutaneous permeation of 5-aminolevulinic acid)

### <Preparation of Formulation>

In a 5 mL-volume glass vial bottle was weighed 4 mg of 5-aminolevulinic acid (5-ALA), 4 mL of isopropyl myristate containing 25 mg/mL of MGOL-70 was added thereto, and the mixture was stirred, by using a polytron homogenizer PT2500 (manufactured by Kinematika) equipped with a 7 mm diameter shaft, at 15,000 rpm for 1 minute to obtain an S/O-S formulation containing 1 mg/mL 5-ALA.

On the other hand, 1 mg of 5-ALA dissolved in pure water and 20 mg of sucrose erucic acid ester ER-290 dissolved in cyclohexane were added to a glass vial. The oil phase and the aqueous phase were stirred, by using a polytron homogenizer PT2500 (manufactured by Kinematika) equipped with a 12 mm diameter shaft, at 26,000 rpm for 2 minutes to prepare a Water-in-oil (W/O) emulsion. This W/O emulsion was lyophilized and the obtained paste state surfactant-drug complex was dispersed in 1 mL of IPM to obtain an S/O formulation containing 1 mg/mL 5-ALA, which was used as a control.

In 1 mL of a PBS solution was dissolved 1 mg of 5-ALA, to provide a PBS solution containing 1 mg/mL 5-ALA as a control.

### <In vitro skin permeability test>

The subcutaneous tissue of the skin of HR-1 mouse purchased from Hoshino Laboratory Animals Inc. was removed and the resultant skin was set in a Franz-type diffusion cell (effective area 0.785 cm²), and its receiver phase was filled with 5 mL of PBS solution. To its donor phase was added 200 µL of the S/O-S preparation and it was incubated at 32°C. After 5 hours, the skin was recovered, washed with ethanol and pure water (Milli-Q water), divided into four parts and shaken at high speed in 1 mL of PBS. The amount of the 5-aminolevulinic acid extracted in the PBS was quantified by using LC-MS.

Tables 5 and 6 show the results of the skin permeability test. In the case of the S/O-S formulation, the 5-aminolevulinic acid could be percutaneously permeated with a 6-times higher efficiency than the PBS solution.

**[Table 5]**

| 5-ALA amount in skin | Average amount ( *µ* g/cm²) | Standard deviation |
|---|---|---|
| 5-ALA PBS | 1.90 | 1.69 |
| S/0 | 0.99 | 0.16 |
| s/0-S | 11.80 | 3.91 |

**[Table 6]**

| 5-ALA amount in receiver | Average amount ( *µ* g/cm²) | Standard deviation |
|---|---|---|
| 5-ALA PBS | n.d. | n.d. |
| S/0 | n.d. | n.d. |
| s/0-S | 67.40 | 38.46 |

### (Example 11: Effect of fatty acid ester of glycerin on skin permeability of ovalbumin (OVA))

### <Preparation of Formulation>

In 5 mL-volume glass vial bottle was weighed 8 mg of OVA labeled with FITC (Fluorescein isothiocyanate), and 4 mL of IPM was added thereto. They were stirred, by using a polytron homogenizer PT2500 (manufactured by Kinematika) equipped with a 7 mm diameter shaft, at 20,000 rpm for 2 minutes to obtain Formulation 1.

Glyceryl monooleate and glyceryl monolinoleate were dissolved in IPM with the composition shown in the following Table 7 to prepare Formulation 2.

Formulation 1 and Formulation 2 were mixed with a volume ratio of 1:1, and used in the following skin permeability test.

**[Table 7]**

| | Ratio of glyceryl monooleate/glyceryl monolinoleate | | | | |
|---|---|---|---|---|---|
| | 100/0 | 90/10 | 75/25 | 50/50 | 0/100 |
| Glyceryl monooleate (mg) | 50 | 45 | 37.5 | 25 | 0 |
| Glyceryl monolinoleate (mg) | 0 | 5 | 12.5 | 25 | 50 |
| I PM (mL) | 1 | 1 | 1 | 1 | 1 |

### <In vitro skin permeability test>

The subcutaneous tissue of the skin of HR-1 mouse purchased from Hoshino Laboratory Animals Inc. was removed and the resultant skin was set in a Franz-type diffusion cell (effective area 0.785 cm²), and its receiver phase was filled with 5 mL of PBS solution. To its donor phase was added 200 µL of the S/O-S preparation and it was incubated at 32°C. After 4 hours, the receiver phase was recovered, and the amount of the FITC-OVA permeated through the skin was quantified by using a plate reader. The results are shown in Table 8.

The relationship between glyceryl monolinoleate and the amount of skin permeation in Table 8 shows that, accompanying with increase in the addition ratio of glyceryl monolinoleate, the skin permeation amount of the drug was increased.

**[Table 8]**

| | Ratio of glyceryl monooleate/glyceryl monolinoleate | | | | |
|---|---|---|---|---|---|
| | 100/0 | 90/10 | 75/25 | 50/50 | 0/100 |
| Average skin (*µ*g/cm²) | 0.22 | 0.91 | 0.44 | 1. 42 | 2.46 |
| SD | 0.07 | 0.83 | 0.13 | 0.72 | 0.36 |

### SEQUENCE LISTING

### FP4435PCT ST25.txt

## Claims

1. A transdermal formulation containing a hydrophilic drug and an oily base, **characterized in that** at least a part of the hydrophilic drug exists in a suspended state with no dissolution in the oily base.

2. The transdermal formulation according to Claim 1, which further comprises an oil-soluble percutaneous permeation enhancing substance.

3. The transdermal formulation according to Claim 2, wherein the oil-soluble percutaneous permeation enhancing substance is at least one kind selected from the group consisting of phosphoglycerides, glycerides, fatty acid esters of sugar alcohols, fatty acid esters of glycols, fatty acids, terpenes and essential oils.

4. The transdermal formulation according to Claim 2 or 3, wherein the oil-soluble percutaneous permeation enhancing substance is a terpene.

5. The transdermal formulation according to Claim 2 or 3, wherein the oil-soluble percutaneous permeation enhancing substance is a fatty acid ester of glycerin.

6. The transdermal formulation according to Claim 5, wherein the oil-soluble percutaneous permeation enhancing substance is an unsaturated fatty acid ester of glycerin.

7. The transdermal formulation according to Claim 6, wherein a carbon chain of the unsaturated fatty acid is 16 or more and 22 or less.

8. The transdermal formulation according to any one of Claims 2, 3 and 5 to 7, wherein the oil-soluble percutaneous permeation enhancing substance is glyceryl monooleate.

9. The transdermal formulation according to any one of Claims 1 to 8, wherein a molecular weight of the hydrophilic drug is 500 Da to 200 kDa.

10. The transdermal formulation according to any one of Claims 1 to 9, wherein a zeta potential of the hydrophilic drug is -50 to 10 mV.

11. The transdermal formulation according to any one of Claims 1 to 10, which contains substantially no water.

12. A method of improving percutaneous absorbability of a hydrophilic drug **characterized in** making, in a transdermal formulation containing the hydrophilic drug and an oily base, at least a part of the hydrophilic drug into a suspended state with no dissolution in the oily base.

13. The method according to Claim 12, wherein the transdermal formulation further contains an oil-soluble percutaneous permeation enhancing substance.

14. The method according to Claim 13, wherein the oil-soluble percutaneous permeation enhancing substance is at least one kind selected from the group consisting of phosphoglycerides, glycerides, fatty acid esters of sugar alcohols, fatty acid esters of glycols, fatty acids, terpenes and essential oils.

15. The method according to Claim 13 or 14, wherein the oil-soluble percutaneous permeation enhancing substance is a terpene.

16. The method according to Claim 13 or 14, wherein the oil-soluble percutaneous permeation enhancing substance is a monofatty acid ester of glycerin.

17. The method according to Claim 16, wherein the oil-soluble percutaneous permeation enhancing substance is an unsaturated fatty acid ester of glycerin.

18. The method according to Claim 17, wherein a carbon chain of the unsaturated fatty acid is 16 or more and 22 or less.

19. The method according to any one of Claims 13, 14 and 16 to 18, wherein the oil-soluble percutaneous permeation enhancing substance is glyceryl monooleate.

20. The method according to any one of Claims 12 to 19, wherein a molecular weight of the hydrophilic drug is 500 Da to 200 kDa.

21. The method according to any one of Claims 12 to 20, wherein a zeta potential of the hydrophilic drug is -50 to 10 mV.

22. The method according to any one of Claims 12 to 21, which the transdermal formulation contains substantially no water.

23. A method for producing a transdermal formulation, which comprises a step of mixing a hydrophilic drug and an oily base and a step of precipitating at least a part of the hydrophilic drug.
